# EUROPEAN PATENT APPLICATION

(11) **EP 3 029 037 A1**
(43) Date of publication of application: **08.06.2016**
(21) Application number: 14196660.6
(22) Date of filing: 05.12.2014
(51) Int. Cl.: C07D 401/14, C09K 11/06, H01L 51/50

(54) **Acridine derivatives and their use in organic electronic devices**

(71) Applicant: Solvay SA, 1120 Bruxelles (BE)
(72) Inventor: Bascour, Dominique, 1390 Grez-Doiceau (BE); Marchand, Dominique, 4671 Saive (BE); Maunoury, Jonathan, 1030 Brussels (BE)
(74) Representative: Dr. Langfinger & Partner

(57) **Abstract**

Acridine derivatives of general formula III 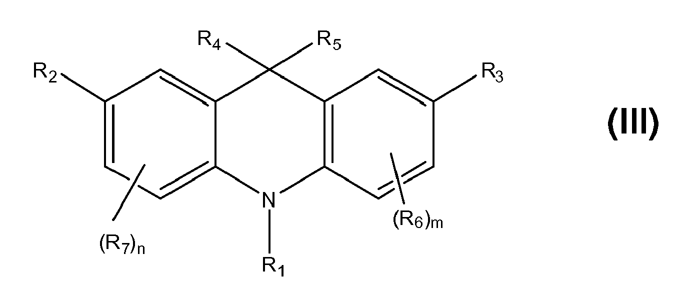 wherein at least one of R₂ and R₃ is represented by formula IV,

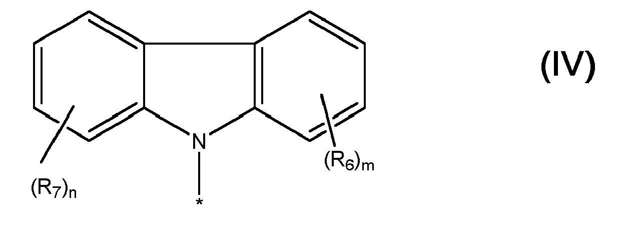

## Description

The present invention relates to new acridine derivatives and their use on organic electronic devices.

Organic light-emitting diodes (OLEDs) exploit the property of materials of emitting light when they are excited by electrical current. OLEDs are of particular interest in this regard as an alternative to cathode ray tubes and to liquid-crystal displays for producing flat visual display units.

Due to the compact design and the intrinsically low power consumption compared to classical lighting devices, devices comprising OLEDs are suitable especially for mobile applications, for example for applications in mobile phones, lap-tops, digital cameras, and for illumination purposes.

The basic principles of the way in which OLEDs work and suitable structures (layers) of OLEDs are known to those skilled in the art.

The light-emitting materials (emitters) may be fluorescent materials (fluorescence emitters) or phosphorescent materials (phosphorescence emitters). The phosphorescence emitters have the advantage over fluorescent emitters that they exhibit triplet emission whereas fluorescence emitters only exhibit singlet emission. As a consequence, the quantum efficiency, energy efficiency and power efficiency of devices using phosphorescent emitters may be up top four times as high than for fluorescence emitters.

In order to implement the advantages of the use of the organometallic triplet emitters in practice, it is necessary to provide device compositions which have a high operative lifetime, a high stability to thermal stress and a low use and operating voltage.

Usually, organic light emitting devices comprise several layers to optimize the suitability of the device for the specific intended purpose.

The emissive layer comprises the emissive material and, in many cases a matrix material in which the emissive material is distributed. This material is generally referred to as host material.

Besides the emissive layer organic electronic devices usually comprise so called hole transport layers (HTL), sometimes also referred to as electron blocking layers.

Certain acridine derivatives have been described in the prior art as host materials as well materials for HTL/EBL layers.

US 2010/0219406 discloses the of acridine derivatives as matrix materials and /or electron blockers in OLEDs. The acridine derivatives are characterized by general formula I 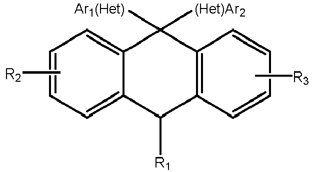 in which HetAr₁ respectively HetAr₂ are each independently an unsubstituted or substituted aryl radical or an unsubstituted or substituted heteroaryl radical,
R₁ is an unsubstituted or substituted alkyl radical which may be linear or branched, an unsubstituted or substituted cycloalkyl radical, an unsubstituted or substituted heterocycloalkyl radical, an unsubstituted or substituted aryl radical, or an unsubstituted or substituted heteroaryl radical, and
R₂ and R₃ are each independently an unsubstituted or substituted alkyl radical which may be linear or branched, an unsubstituted or substituted cycloalkyl radical, an unsubstituted or substituted heterocycloalkyl radical, an unsubstituted or substituted aryl radical, an unsubstituted or substituted heteroaryl radical, or a silyl radical.

US 2012/0319052 also discloses acridine derivatives for use in organic electronic devices. The compounds comprise an acridine unit with a substituent attached to the nitrogen atom which may be an aryl or a heteroaryl group, which aryl or heteroaryl group is further substituted by a carbazolyl group, which may be attached via the carbazole nitrogen or via a carbon atom of the phenyl rings of the carbazole structure.

US 2009/066226 discloses electroluminescent devices comprising compounds of general formula II as constituent of an emissive layer 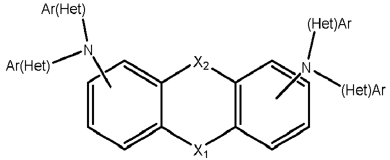 wherein Het(Ar) is an aromatic or a heteroaromatic ring with the possibility of the two rings attached to a nitrogen atom being linked through a divalent substituent or through a chemical bond, X₁ inter alia being an amino group and X₂ inter alia being an alkylene group. The phenyl rings of the acridine core may bear further substituents. The nitrogen containing substituent is i.a. a carbazolyl group in certain preferred embodiments.

WO 2006/033563 discloses similar compounds of general formula 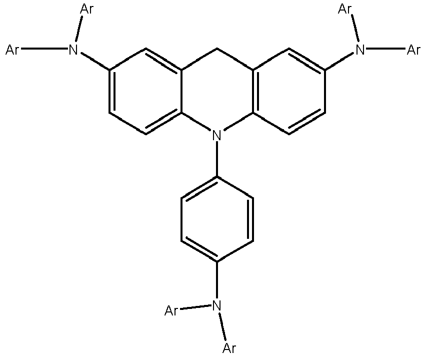 wherein the N(Ar)₂ substituents may represent a carbazole group.

The acridine compounds disclosed in the prior art are not fully satisfactory in terms of performance when used in organic electronic devices.

It was thus an object of the present invention to provide new compounds useful as host materials in the emissive layers of organic electronic devices or as constituent materials in other layers of organic electronic devices which show a good efficiency and stability in these devices and overcome the deficiencies of the prior art materials at least partly.

This object has been achieved with the acridine derivatives of general formula III as defined in claim1.

Further preferred embodiments of the present invention are set forth in the dependent claims and the detailed specification hereinafter.

The acridine derivatives in accordance with the present inventions are represented by general formula (III) 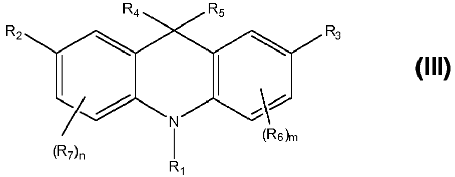 wherein R₁ is a C₁-C₁₈ alkyl group, a C₅-C₃₀ aryl group or a C₄-C₃₀ heteroaryl group wherein the aryl or heteroaryl group may be unsubstituted or substituted by substituents selected from the group consisting of halogen, alkyl, alkoxy, cyano, alkenyl, alkinyl and arylalkyl, groups,
R₄ and R₅, which may be the same or different at each occurrence, are hydrogen, a C₁-C₁₈ alkyl group, a C₅-C₃₀ aryl group or a C₄-C₃₀ heteroaryl group,
R₂ and R₃, which may be the same or different at each occurrence, are a C₁-C₁₈ alkyl group, a C₅-C₃₀ aryl group or a C₄-C₃₀ heteroaryl group or a group NR₈R₉ wherein R₈ and R₉, which may be the same or different at each occurrence, are a C₁-C₁₈ alkyl group, a C₅-C₃₀ aryl group or a C₄-C₃₀ heteroaryl group, which may form together a condensed ring system,

or wherein R₂ and R₃, which may be the same or different, represent a group of formula (IV),

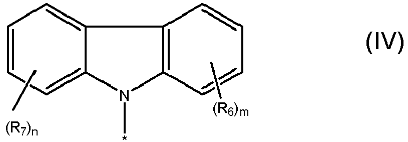

R₆ and R₇, which may be the same or different at each occurrence, are a C₁-C₁₈ alkyl group, a C₅-C₃₀ aryl group or a C₄-C₃₀ heteroaryl group or a group NR₈R₉ wherein R₈ and R₉, which may be the same or different at each occurrence are a C₁-C₁₈ alkyl group, a C₅-C₃₀ aryl group or a C₄-C₃₀ heteroaryl group or wherein NR₈R₉ is represented by formula IV, and m and n, which may be the same or different, represent an integer of from 0 to 3.

At least one of R₂ and R₃ is represented by formula IV which means that the compounds in accordance with the present invention comprise at least one carbazole type group and one acridine type group.

In accordance with a first preferred embodiment of the present invention, the acridine derivatives are represented by general formula (V) 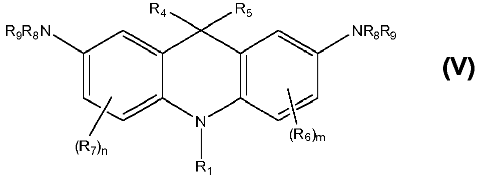 wherein R₁ and R₄ to R₉ are as defined above.

Acridine derivatives of formula (V) wherein both groups NR₈R₉, which may be the same or different, are represented by formula (IV) are a further preferred embodiment of the present invention.

Acridine derivatives wherein n and m are 0 represent a further preferred group of compounds in accordance with the present invention.

In accordance with still another embodiment at least one of R₄ and R₅ is an alkyl group.

Acridine derivatives wherein R₁ is a C₅-C₃₀ aryl group or a C₄-C₃₀ heteroaryl group, more preferably a C₅-C₃₀ aryl group and especially preferably a phenyl group represent another embodiment of the present invention.

A particularly preferred group of compounds in accordance with the present invention is represented by the general formula (VI) wherein R₁, R₄ and R₅ are as defined above.

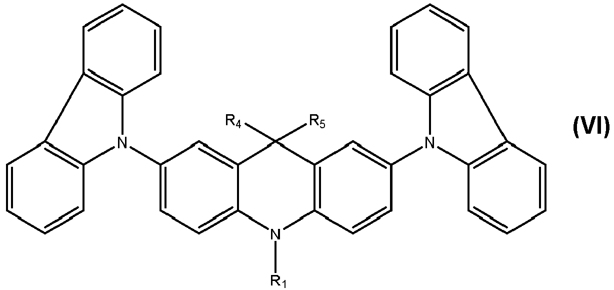

As outlined above for compounds of formulas (III) to (V), R₄ and R₅ are preferably an alkyl group and R₁ is preferably an aryl group, especially preferably a phenyl group.

The term C₁-C₁₈ alkyl group, as used herein, denotes a linear, branched or cyclic group comprising 1 to 18 carbon atoms with hydrogen atoms as substituents. Linear or branched alkyl groups having 1 to 12, in particular 1 to 8 carbon atoms are preferred. Linear alkyl groups having 1 to 6 and especially 1 to 4 carbon atoms such as methyl, ethyl, n-propyl, n-butyl, n-pentyl and n-hexyl are especially preferred.

Representative examples of branched alkyl groups with 1 to 8 carbon atoms are i-propyl, i- and t-butyl and the isomeric branched methyl-or ethyl-substuted pentyl- or hexyl derivatives like e.g. 2-methylpentyl, 3-methylpentyl, 2-ethylhexyl, 3-ethylhexyl or 4-ethylhexyl to mention only a few typical representatives.

The term C₅-C₃₀ aryl group, for the purposes of the present invention denotes groups having at least one 5 to 7 membered aromatic ring with (4n+2) *π* electrons in accordance with the so called Hückel rule. Two or more aromatic rings may be annealed of fused or may be connected through a bond or an alkyl group with each other.

Representative aryl groups are thus phenyl, biphenyl, naphthyl or anthracenyl.

The aromatic rings in the aryl groups may be un-substituted or substituted by substituents selected from the group consisting of halogen, alkyl, alkoxy, cyano, alkenyl, alkynyl and arylalkyl groups.

Particularly preferred aryl groups are C₆-C₁₄ aryl groups like phenyl, biphenyl naphthyl and anthracenyl, which may be substituted or unsubstituted and in some cases substituted or unsubstituted phenyl, in particular unsubstituted phenyl have shown advantageous results.

The term C₄-C₃₀ heteroaryl group, when used herein, denotes a group having an aromatic ring comprising at least one heteroatom, which may be substituted or unsubstituted as described above for the aryl groups.

Preferred heteroaryl groups are heteroaryl ring containing at least one donor nitrogen atom. Said ring may be un-substituted or substituted by substituents selected from the group consisting of halogen, alkyl, alkoxy, amino, cyano, alkenyl, alkynyl, arylalkyl, aryl and heteroaryl group and/or may form an annealed ring system with other rings selected from cycloalkyl, aryl and heteroaryl rings. Heteroaryl substituents may be preferably un-substituted or substituted carbazolyl or un-substituted or substituted dibenzofuranyl.

More particularly heteroaryl groups are derived from the heteroarenes group consisting of 2H-pyrrole, 3H-pyrrole, 1H-imidazole, 2H-imidazole, 4H-imidazole,1H-1,2,3-triazole, 2H-1,2,3-triazole, 1H-1,2,4-triazole, 1H-pyrazole, 1H-1,2,3,4-tetrazole, imidazol-2-ylidene, oxazole, isoxazole, thiazole, isothiazole, 1,2,3-oxadiazole, 1,2,5-oxadiazole, 1,2,3-thiadiazole and 1,2,5-thiadazole rings.

Nitrogen containing heteroaryl groups are preferably derived from the heteroarenes shown below 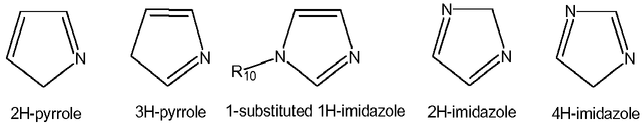 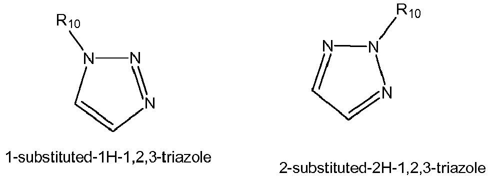 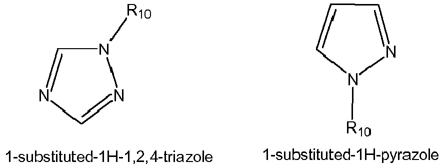 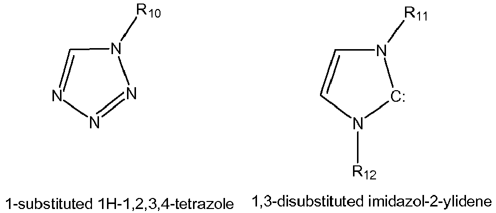 wherein R₁₀, R₁₁ and R₁₂ may be selected from a broad variety of substituents such as alkyl, alkenyl, alkynyl, arylalkyl, aryl and heteroaryl groups.

Particularly preferred acridine compounds in accordance with the present invention ar the compounds of formula (VI) wherein R₄ and R₅ represent a C₁-C₄ alkyl group, especially a methyl group and R₁ represents a C₆-C₁₄ aryl group, particularly preferred a phenyl group.

Especially preferred compounds of formula (VI) are the following:

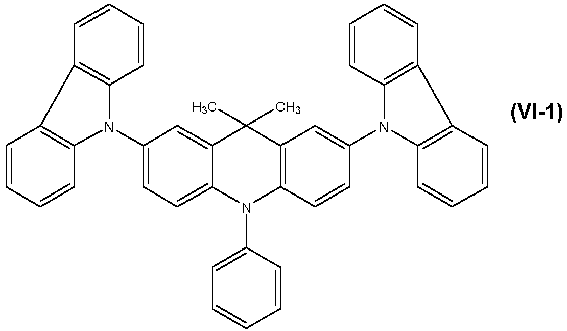

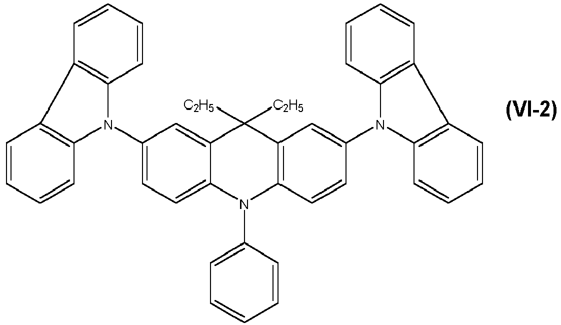

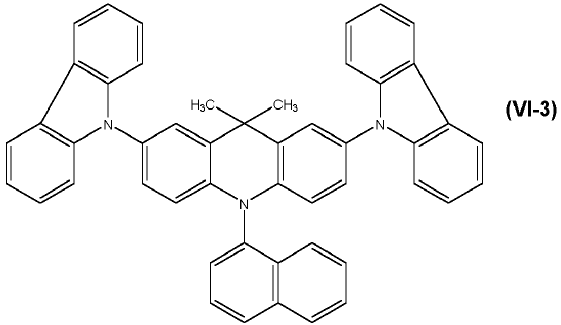

The aryl group at the nitrogen atom of the central acridine unit may be unsubstituted or substituted as described above.

The acridine compounds in accordance with the present invention may be obtained in accordance with synthetic methods which are known per se and of which the skilled person is aware.

A preferred process for the manufacture of the compounds in accordance with the present invention is described hereinafter for the most preferred compounds of formula VI described above; the skilled person will modify the starting materials and reactants appropriately to synthesize other compounds in accordance with the present invention, i.e. the process described is just exemplary show for specific compounds but is generally applicable.

The starting material is an acridine compound comprising substituents R₄ and R₅ as described hereinbefore and in the scheme below both R₄ and R₅ are methyl.

In a first reaction step, the hydrogen atom at the acridine nitrogen is replaced by the desires substituent R₁, in the scheme exemplified for R₁ being phenyl. This is achieved by reacting the acridine compound with a compound R₁-Hal, in particular R₁-Br as shown in the scheme in the presence of suitable catalysts and in a suitable solvent. Just by way of example toluene may be used as a solvent, and a combination of Pd(dba)₂ (Bis(dibenzylideneacetone) palladium(0)) P(t-bu)₃ (tris tert. butyl phosphine) and sodium tert. butanoate may be used as catalyst system. The skilled person is aware of this type of reaction and will appropriately select other catalyst systems or solvents as needed for the manufacture of the compounds in accordance with the present invention so that no further details concerning the reaction components need to be given here.

The first reaction step is usually carried out under inert atmosphere conditions, i.e. with the exclusion of oxygen and the reaction mixture after adding all components is heated to a temperature in the range of from 70 to 150°C, preferably of from 90 to 130°C to start the reaction. In case of toluene as solvent, the reaction may be carried at reflux. The reaction time is usually in the range of from 6 to 48h, preferably of from 9 to 36 hours, depending on the reactants used in the specific case.

After complete conversion the reaction mixture is preferably filtered (e.g. through a celite pad) and the solvent is removed under vacuum. The purification may be carried out by chromatography, in particular flash chromatography. The desired product, bearing the substituent R₁ at the acridine nitrogen atom is usually obtained in high yields exceeding 50, in many cases exceeding seventy and in many cases even exceeding 90 %.

The reaction scheme of the first reaction step is shown below:

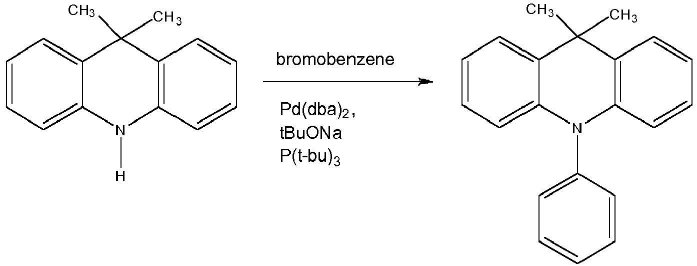

In the second step, the phenyl rings of the acridine core are brominated to create a intermediate to which the substituents R₂ and R₃ are attached in the final step.

Again, respective processes are known to the skilled person and will be selected in accordance to the needs of a specific final product, so that no further details need to be given here.

Just by way of example N-bromo succinimide may be mentioned as common brominating agent which may be used together with a suitable catalyst, e.g. Fe(III)chloride. The reaction temperature with this system is usually in the range of from 0 to 70°C, preferably of from 10 to 50°C and even more preferably around room temperature (20 to 30°C). The reaction time is usually in the range of from 6 to 96 h, preferably of from 12 to 60 hours and even more preferably in the range of from 18 to 48 h at room temperature. The skilled person will adopt the reaction conditions dependent on the starting material and the bromination agent used.

The exemplary reaction described above is represented below in a reaction scheme:

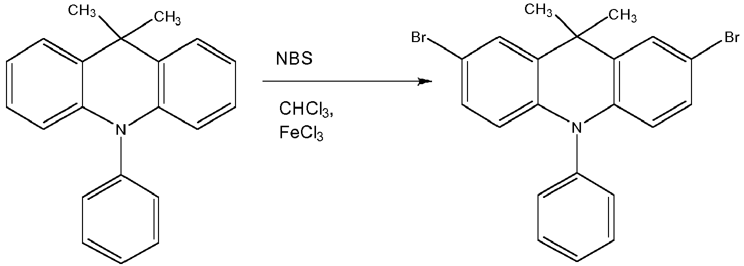

In the final reaction step, the compounds in accordance with the present invention are then obtained by substituting the bromine atoms at the phenyl rings of the acridine by R₂ respectively R₃. The respective substitution reactions suitable for this step have been described in the literature so that no further details need to be given here.

Just by way of example, the reaction conditions may be similar as in the first step, i.e. a catalyst system comprising Pd(dba)₂ and P(t-bu)₃ with a combination of toluene and NaOt-Bu may be used.

The molar ratio of the reactants in the various steps is chosen in accordance with the stoichiometry of the reaction. In the first step it has proved to be advantageous to use the brominating agent in a slight molar excess, e.g. 5 to 20 %, compared to the acridine derivative. The same applies to the second step.

The amount of catalyst is usually in the range of from 0.5 to 5 mol%, based on the brominating agent respectively starting material, but can be varied in accordance with the specific reaction carried out.

In the final step, the reactants are again basically used in stoichiometric amounts.

The acridine compounds in accordance with the present invention usually have glass transition temperatures in the range of from 120 to 200°C, mostly in the range of from 130 to 180°C.

The acridine compounds in accordance with the present invention are suitable for use in organic electronic devices, in particular organic light emitting devices (OLED.

An OLED generally comprises:
a substrate, for example (but not limited to) glass, plastic, metal;
an anode, generally transparent anode, such as an indium-tin oxide (ITO) anode;
a hole injection layer (HIL) for example (but not limited to) PEDOT/PSS;
a hole transporting layer (HTL);
an emissive layer (EML);
an electron transporting layer (ETL);
an electron injection layer (EIL) such as LiF, Cs₂CO₃ a cathode, generally a metallic cathode, such as an Al layer. For a hole conducting emissive layer, one may have a hole blocking layer (HBL) that can also act as an exciton blocking layer between the emissive layer and the electron transporting layer. For an electron conducting emissive layer, one may have an electron blocking layer (EBL) that can also act as an exciton blocking layer between the emissive layer and the hole transporting layer. The emissive layer may be equal to the hole transporting layer (in which case the exciton blocking layer is near or at the anode) or to the electron transporting layer (in which case the exciton blocking layer is near or at the cathode).

The compounds in accordance with the present invention have appropriate HOMO/LUMO and triplet energy levels to make them suitable e.g. as hosts for phosphorescent emitters in an emissive layer of an organic electronic device, in particular an OLED. Their triplet energy level usually exceeds 2.7 eV and the compounds are thus also useful as host materials for blue phosphorescent emitters.

The acridine compounds in accordance with the present invention are also useful as materials for the electron blocking layer of respective organic light emitting diodes.

When used as hosts in the emissive layer together with an emitter, the performance of the device in terms of light intensity and quantum efficiency is often improved compared to devices with other host materials.

The following examples describe the synthesis of a preferred acridine compound in accordance with the present invention and its performance in OLED devices.

### Example 1

Synthesis of compound of formula (VI) with R₄ and R₅ being methyl and R₁ being phenyl (see also reaction scheme provided above as example for preferred process):

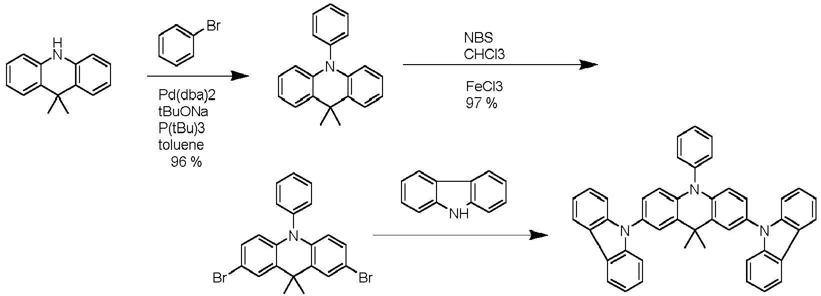

### First step:

In a three ways flask 6.5 g of bromo-benzene (0.042 mol), 8.0 g of dimethyl acridine (0.038 mol) and 11.0 g of sodium ter-butanoate (0.11 mol) were introduced. Everything was kept under inert atmosphere and the solids were dissolved in 190 ml of toluene. The catalyst was prepared in a 50 ml flask, by mixing 870 mg of Pd(dba)2 (1.5 mmol) and 3.0 ml of P(tBu)3 (3 mmol) in 30 ml of toluene. This catalyst solution was added to the reaction medium and the medium was warmed for one night at reflux. After complete conversion, the reaction medium was filtrated on a celite pad (diatomaceous earth), and the solvent was removed under vacuum. Purification using flash chromatography (CH₂Cl₂/hexane) afforded 10.6 g of target compound (N-Phenyl-dimethylacridine) in 96 % yield.

### Second step: Bromination of reaction product of first step

The bromination was realized using 2.1 equivalents of NBS (N-Bromosuccinimide, 13.7 g, 0.076 mol) in CHCl₃ (420 mL) with the acridine previously synthesized (10.6 g, 0.036 mol). A catalyst was added to the reaction medium (FeCl₃.6H₂O, 0.06 eq, 0.6 g, 0.002 mol) and mixed during 24 hours at room temperature. After usual work-up and flash chromatography on silica gel (CH₂Cl₂/hexane), 16.0 g of product (dibrominated N-phenyl dimethyl acridine) was obtained in a yield of 97 %.

### Third step

In a 500 mL three ways flask, 6.6 g of Dibromo-Dimethyl-acridine obtained in the second step (0.014 mol), 4.8 g of carbazole (2.03 eq, 0.029 mol) and 16.6 g of NaOtBu (5 eq, 0.072 mol) were introduced. 160 mL of anhydrous toluene were added and the whole medium was let 30 minutes under inert atmosphere. The catalyst was prepared in a 50 ml flask, by mixing 665 mg of Pd(dba)2 (1.2 mmol) and 2.3 ml of P(tBu)3 (2.3 mmol) in 25 ml of toluene. This catalyst solution was added to the reaction medium and warmed for one night at reflux. At the end of the reaction the medium was washed with water, and the organic layer was evaporated to afford a brown solid. This solid was washed in 400 mL of warm hexane to afford the target compound as a white product with 88 % yield (9.7 g). The triplet energy was determined to be 2.97 eV and the glass transition temperature was 158°C.

### Example 2: Performance of compound of Example 1 as host in the emissive layer of an OLED

### The device was fabricated by high vacuum thermal evaporation, except for the hole injecting layer which was deposited by the spin-coating technique. The anode electrode was 120 nm of indium tin oxide (ITO). All devices were encapsulated with a glass lid sealed with an epoxy resin in a nitrogen glovebox (<1 ppm of H₂O and O₂) immediately after fabrication, and a moisture getter was incorporated inside the package. The devices were characterized optically and electrically with a C9920-12 External Quantum Efficiency Measurement System from HAMAMATSU Photonics. EQE refers to external quantum efficiency expressed in %, while PE refers to power efficiency expressed in lm/W and CIE refers to the 1931 Commission Internationale de l'Eclairage (CIE) coordinates. CE stands for the current efficiency in Cd/A while V stands for the Voltage in Volts. Vₒₙ depicts the onset-Voltage.

An OLED with the following layer set-up was prepared by vacuum deposition of the layers.

| |
|---|
| AI (100 nm) |
| Electron injection layer (EIL) : Cs2CO3 (40 nm) |
| Electron transport layer (ETL) (10 nm) |
| Emitting layer (EML): Host: 30% Blue emitter (30 nm) |
| Hole transporting layer (HTL) (15 nm) |
| Hole injection layer (HIL) (15 - 20 nm) |
| ITO / Glass |

The OLED stack consisted of sequentially, from the ITO surface which formed the anode, a hole injection layer of 15-20 nm of Plexcore^{®} OC AQ (a self-doping polymer poly(thiophene-3-[2[(2-methoxyethoxy)ethoxy]-2,5-diyl), supplied by Plextronics Inc.) deposited by spin-coating and dried on a hot plate under inert atmosphere at 180 °C for 20 min. On top of the HIL, 30 nm of NPB were deposited by vacuum-thermal evaporation as hole transporting layer (HTL).

Then a 30 nm layer of Compound A (Comparative Example) or the compound of Example 1, in both cases doped with 30% of Compound B as an emitter was deposited by vacuum-thermal evaporation as the emissive layer (EML). Then a 5nm layer of compound A was deposited by vacuum-thermal evaporation as the hole blocking layer (HBL), also referred to as electron transporting layer (ETL). Then, a 40 nm layer of Cs₂CO₃ was deposited by vacuum-thermal evaporation as the electron injecting layer (EIL). The cathode consisted of 100 nm of Aluminum.

NPB, Compound A and Compound B have the following structures:

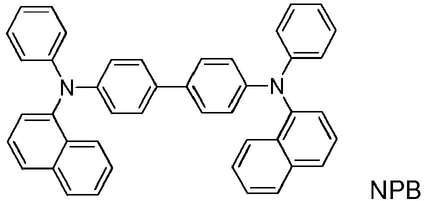

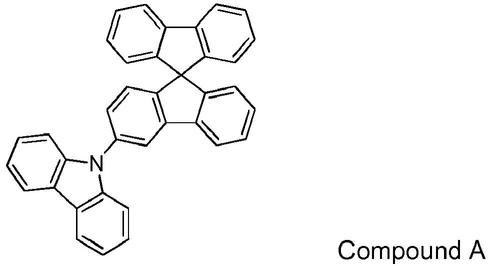

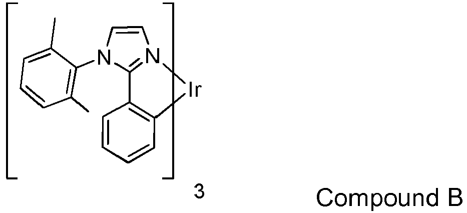

Table 1 shows the results measured at 1000 cd/m² for the fabricated devices:

**Table 1**

| **Example** | **Host in EML.** | **V** | **EQE %** | **PE lm/W** | **CE Cd/A** | **CIE X** | **CIE Y** | **Vₒₙ** |
|---|---|---|---|---|---|---|---|---|
| Comp. Ex. | Comp A | 3,6 | 13.2 | 30,5 | 34.5 | 0,21 | 0,46 | 2,6 |
| Ex. 2 | Cpd. Ex. 1 | 4.1 | 16.5 | 33.4 | 43.8 | 0.22 | 0.47 | 3.0 |

It can easily be seen that replacing compound A as host of the emissive layer by the compound of Example 1 significantly increases the performance of the device in terms of quantum efficiency (EQE), power efficiency (PE) and current efficiency (CE) without detrimentally influencing other properties of the device.

### Example 3: Performance of the compound of Example 1 as material for the electron blocking layer of an OLED

The OLED had the same principal set-up as the device of Example 2 with the difference that a HBL layer consisting of the compound of Example 1 with a thickness of 5 nm was deposited between the HTL and the emissive layer while in the comparative device this additional layer was not present

| |
|---|
| AI (100 nm) |
| Electron injection layer (EIL) :Compound A Cs2CO3 (40 nm) |
| Electron transport layer (ETL): Compound A (10 nm) |
| Emitting layer (EML): Compound A : 30% Blue emitter (30 nm) |
| Hole transporting layer (HTL) (15 nm) |
| Hole injection layer (HIL) (15 - 20 nm) |
| ITO / Glass |

By the insertion of an electron blocking layer consisting of the compound of example 1 the electrical quantum efficiency (EQE), the power efficiency (PE) and the current efficiency (CE) increased by 25 %, 10% and 28% , respectively without detrimental influence on other parameters. This example shows that the use of the compounds in accordance with the present invention as materials of an electron blocking layer improve the performance of the device compared to devices without such layers.

## Claims

1. Acridine derivatives of general formula III 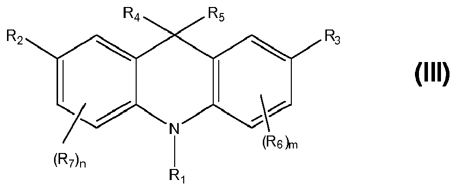 wherein R₁ is hydrogen, a C₁-C₁₈ alkyl group, a C₅-C₃₀ aryl group or a C₄-C₃₀ heteroaryl group wherein the aryl or heteroaryl group may be unsubstituted or substituted by substituents selected from the group consisting of halogen, alkyl, alkoxy, cyano, alkenyl, alkinyl and arylalkyl groups,
R4 and R5, which may be the same or different at each occurrence, are a C₁-C₁₈ alkyl group, a C₅-C₃₀ aryl group or a C₄-C₃₀ heteroaryl group, 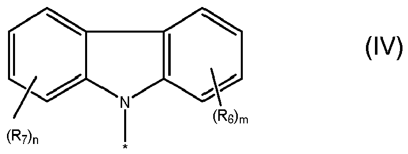 R₂ and R₃, which may be the same or different at each occurrence, are a C₁-C₁₈ alkyl group, a C₅-C₃₀ aryl group or a C₄-C₃₀ heteroaryl group or
a group NR₈R₉ wherein R₈ and R₉, which may be the same or different at each occurrence, are a C₁-C₁₈ alkyl group, a C₅-C₃₀ aryl group or a C₄-C₃₀ heteroaryl group, which may form together a condensed ring system,
or wherein R₂ and R₃, which may be the same or different at each occurrence, represent a group of formula (IV),
R₆ and R₇, which may be the same or different at each occurrence, are a C₁-C₁₈ alkyl group, a C₅-C₃₀ aryl group or a C₄-C₃₀ heteroaryl group or a group NR₈R₉ wherein R₈ and R₉, which may be the same of different at each occurrence, are a C₁-C₁₈ alkyl group, a C₅-C₃₀ aryl group or a C₄-C₃₀ heteroaryl group or wherein NR₈R₉ is represented by formula IV,
and m and n, which may be the same or different, represent an integer of from 0 to 3, with the proviso that at least one of R₂ and R₃ is represented by formula IV.

2. Acridine derivatives in accordance with claim 1 represented by formula V 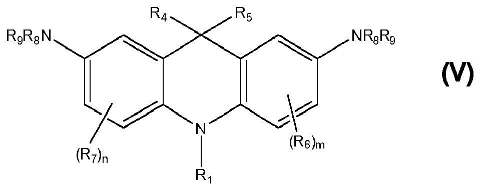 wherein R₁ and R₄ to R₉ are as defined in claim 1.

3. Acridine derivatives in accordance with claim 2 wherein both groups NR₈R₉, which may be the same or different at each occurrence, are represented by formula (IV).

4. Acridine derivatives in accordance with any of claims 1 to 3 wherein n and m are 0.

5. Acridine derivatives in accordance with any of claims 1 to 4 wherein at least one of R₄ and R₅ is an alkyl group.

6. Acridine derivatives in accordance with any of claims 1 to 5 wherein R₁ is a C₅-C₃₀ aryl group or a C₄-C₃₀ heteroaryl group.

7. Acridine derivatives in accordance with claim 6 wherein R₁ is a phenyl group.

8. Acridine derivatives in accordance with any of claims 1 to 7 represented by formula VI 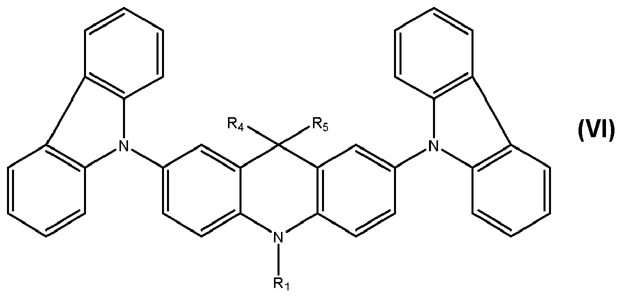 wherein R₁, R₄ and R₅ are as defined in claim 1.

9. The compound in accordance with claim 8 wherein R₁ is phenyl and R₄ and R₅ is methyl.

10. Organic electronic device comprising a compound as defined in any of claims 1 to 9 in at least one layer of an organic electronic device.

11. Organic electronic device in accordance with claim 10 which is an organic light emitting diode.

12. Organic light emitting diode in accordance with claim 11 comprising a compound as defined in any of claims 1 to 9 as host material in the emissive layer and/or in the electron blocking layer.

13. Use of compounds in accordance with any of claims 1 to 9 in at least one layer of an organic electronic device.

14. Use in accordance with claim 13 wherein the organic electronic device is an organic light emitting diode.
